# EUROPEAN PATENT APPLICATION

(11) **EP 3 409 117 A1**
(43) Date of publication of application: **05.12.2018**
(21) Application number: 17744151.6
(22) Date of filing: 24.01.2017
(51) Int. Cl.: A01N 53/06, A01M 1/20, A01N 25/18, A01P 7/02, A01P 7/04, A61L 9/02

(54) **PEST CONTROL PRODUCT AND PEST CONTROL METHOD**

(30) Priority: 25.01.2016 JP 2016011736
(71) Applicant: DAINIHON JOCHUGIKU CO., LTD., Osaka-shi Osaka 550-0001 (JP)
(72) Inventor: ITANO Taisuke, Toyonaka-shi Osaka 561-0827 (JP); ICHIMURA Yumiko, Toyonaka-shi Osaka 561-0827 (JP); KASHIMA Seiichi, Toyonaka-shi Osaka 561-0827 (JP); NAKAYAMA Koji, Toyonaka-shi Osaka 561-0827 (JP)
(74) Representative: Cabinet Beau de Loménie
(86) International application number: PCT/JP2017/002202
(87) International publication number: WO 2017/130921

(57) **Abstract**

Provided is an insect pest control product that is applicable to a water-based insecticidal composition and can continue to exert stable performance over a long period of time. The insect pest control product comprises a thermal vaporization/diffusion absorbent wick for vaporizing and diffusing a water-based insecticidal composition containing a pyrethroid insecticidal component having a vapor pressure of 2 × 10⁻⁴ to 1 × 10⁻² mmHg at 30°C, a glycol ether compound and/or glycol compound having a boiling point of 150-300°C, and water. The thermal vaporization/diffusion absorbent wick has an insecticidal component/compound condensation ratio (P₁/P₂) within the range of 1.2-6.0, where the insecticidal component/compound condensation ratio (P₁/P₂) is calculated from a weight ratio (P₁) of the pyrethroid insecticidal component and the glycol ether compound and/or glycol compound contained in the thermal vaporization/diffusion absorbent wick after use, and a weight ratio (P₂) of the pyrethroid insecticidal component and the glycol ether compound and/or glycol compound contained in the water-based insecticidal composition before use.

## Description

### TECHNICAL FIELD

The present invention relates to insect pest control products equipped with a thermal vaporization/diffusion absorbent wick which are used to vaporize and diffuse a water-based insecticidal composition containing a pyrethroid insecticidal component having a relatively high vapor pressure, and insect pest control methods.

### BACKGROUND ART

Among flying insect pest control products for controlling flying insect pests such as mosquitoes are so-called "liquid mosquito killers," which are commercially available. Liquid mosquito killers utilize the technique of putting an absorbent wick in a chemical liquid containing an insecticidal component, allowing the chemical liquid to be absorbed and transported to the top portion of the absorbent wick, and heating the absorbent wick so that the insecticidal component is vaporized and diffused into the atmosphere. Typical insecticidal components for liquid mosquito killers are pyrethroid insecticidal components. Of pyrethroid insecticidal components, allethrin, prallethrin, furamethrin, etc., have been most commonly used, but lately there has been a trend towards using newer components such as transfluthrin and metofluthrin, which have a higher insecticidal activity.

Chemical liquids for use in liquid mosquito killers include kerosene-based formulations (referred to as "oil-based formulations") and water-based formulations. So far oil-based formulations have been used in most of globally available liquid mosquito killers. However, compared to oil-based formulations, water-based formulations have a reduced risk of catching fire, and are easily made more effective in killing insect pests. Therefore, it is expected that the demand for water-based formulations will increasingly grow in future.

Here, the volatility of the chemical liquid is closely related to the characteristics of the absorbent wick. Therefore, in order to improve the performance (e.g., stability, sustainability, etc.) of the insect pest control product, the configuration of the absorbent wick should be adapted for the formulation of the chemical liquid used. When the chemical liquid is a water-based formulation, it is desirable to adapt the specifications of the absorbent wick for the water-based chemical liquid.

In the related art, an example absorbent wick used for a water-based formulation of the chemical liquid is a baked molded article that contains aggregate including phosphorus oxide (see, for example, Patent Document 1). Patent Document 1 discloses an absorbent wick having a specific surface area of 1.0-3.0 m²/g, a liquid absorption ratio of 15-35%, and a liquid absorption speed of 10-25 mm/hour, and indicates that the absorbent wick having these properties is applicable to a water-based formulation of the chemical liquid.

### CITATION LIST

### PATENT LITERATURE

Patent Document 1: Japanese Unexamined Patent Application Publication No. 2000-103704

### SUMMARY OF INVENTION

### TECHNICAL PROBLEM

An ideal chemical liquid for use in a liquid mosquito killer is such that when an absorbent wick is put in the chemical liquid, the chemical liquid rises through the absorbent wick while maintaining the original ratio of components in the chemical liquid, and is then vaporized and diffused from the top portion of the absorbent wick into the air. However, the absorbent wick disclosed in Patent Document 1 is mainly intended to be applied to dl·d-T80-allethrin, bioallethrin, d·d-T80-prallethrin, etc., which are mostly used in oil-based formulations, but not to pyrethroid insecticidal components, such as transfluthrin and metofluthrin, which are suitable for water-based formulations. Therefore, when there is a difference in vaporization and diffusion capability between components of a water-based formulation of the chemical liquid, a component having a lower vaporization and diffusion capability may generally have a higher concentration at the top portion of the absorbent wick, and therefore, the composition fraction of that component in the chemical liquid may relatively increase. It has been found that such a phenomenon depends on the properties of a material for the absorbent wick, the affinity between the absorbent wick and the chemical liquid components, the liquid absorption speed of the absorbent wick, etc., and particularly easily occurs for water-based formulation of the chemical liquid.

Thus, in order to develop a water-based liquid mosquito killer employing a water-based insecticidal composition containing three components, i.e., a pyrethroid insecticidal component, such as transfluthrin or metofluthrin, a surfactant, and water, the behavior of the water-based insecticidal composition in the absorbent wick should be understood in addition to the formulation of the water-based insecticidal composition. However, such a behavior has hardly been studied so far.

With the above problems in mind, the present invention has been made. It is an object of the present invention to focus on the behavior of a chemical liquid in a thermal vaporization/diffusion absorbent wick, and provide an insect pest control product comprising a thermal vaporization/diffusion absorbent wick that can be used to vaporize and diffuse a chemical liquid containing a pyrethroid insecticidal component having a relatively high vapor pressure, the product being capable of continuing to exert stable performance over a long period of time and applicable to a water-based insecticidal composition. It is another object of the present invention to provide an insect pest control method of using such an insect pest control product.

### SOLUTION TO PROBLEM

To achieve the object, the present invention provides an insect pest control product comprising a thermal vaporization/diffusion absorbent wick for vaporizing and diffusing a water-based insecticidal composition containing a pyrethroid insecticidal component having a vapor pressure of 2 × 10⁻⁴ to 1 × 10⁻² mmHg at 30°C, a glycol ether compound and/or glycol compound having a boiling point of 150-300°C, and water, wherein the thermal vaporization/diffusion absorbent wick has an insecticidal component/compound condensation ratio (P₁/P₂) within the range of 1.2-6.0, where the insecticidal component/compound condensation ratio (P₁/P₂) is calculated from a weight ratio (P₁) of the pyrethroid insecticidal component and the glycol ether compound and/or glycol compound contained in the thermal vaporization/diffusion absorbent wick after use, and a weight ratio (P₂) of the pyrethroid insecticidal component and the glycol ether compound and/or glycol compound contained in the water-based insecticidal composition before use.

The pyrethroid insecticidal component is preferably at least one selected from the group consisting of transfluthrin, metofluthrin, and profluthrin.

The glycol ether compound is preferably a diethylene glycol alkyl ether, more preferably diethylene glycol monobutyl ether.

The thermal vaporization/diffusion absorbent wick is preferably a baked wick or a braided wick. When the thermal vaporization/diffusion absorbent wick is a baked wick, the baked wick preferably contains, as raw materials, an inorganic powder, an inorganic binder, and an organic substance. When the thermal vaporization/diffusion absorbent wick is a braided wick, the braided wick preferably has a core member, and a sheath material covering an outer peripheral surface of the core member. The sheath material preferably contains at least one fiber selected from the group consisting of natural fibers, synthetic fibers, and inorganic fibers.

According to the insect pest control product of the present invention, the water-based insecticidal composition contains the appropriate components, and the thermal vaporization/diffusion absorbent wick has the appropriate structure, and the appropriate insecticidal component/compound condensation ratio (P₁/P₂). Therefore, when the water-based insecticidal composition is vaporized and diffused from the thermal vaporization/diffusion absorbent wick, stable vaporization and diffusion performance and high insect killing efficacy can be simultaneously achieved over a long period of time.

To achieve the object, the present invention provides an insect pest control method of using the insect pest control product of any one of the above aspects, comprising:
putting the thermal vaporization/diffusion absorbent wick in the water-based insecticidal composition so that the water-based insecticidal composition is absorbed and transported to a top portion of the thermal vaporization/diffusion absorbent wick, and heating the top portion at 60-130°C so that the pyrethroid insecticidal component is vaporized and diffused into the atmosphere.

According to the insect pest control method of the present invention, the water-based insecticidal composition is thermally vaporized and diffused using the insect pest control product of the present invention, and therefore, stable vaporization and diffusion performance and high insect killing efficacy can be simultaneously achieved over a long period of time.

### DESCRIPTION OF EMBODIMENTS

An insect pest control product and an insect pest control method according to the present invention will now be described. Note that the present invention is in no way limited to embodiments and examples described below.

A water-based insecticidal composition for a liquid mosquito killer (hereinafter simply referred to as a "water-based insecticidal composition") applicable to the insect pest control product of the present invention contains a pyrethroid insecticidal component having a vapor pressure of 2 × 10⁻⁴ to 1 × 10⁻² mmHg at 30°C. Examples of such a pyrethroid insecticidal component include transfluthrin, metofluthrin, profluthrin, empenthrin, terallethrin, meperfluthrin, heptafluthrin, 4-methoxymethyl-2,3,5,6-tetrafluorobenzyl-chrysanthemate, and 4-methoxymethyl-2,3,5,6-tetrafluorobenzyl-2,2-dimethyl-3-(2-chloro-2-trifluoromethylvinyl)cyclopropanecarboxylate. Of them, transfluthrin, metofluthrin, and profluthrin are preferable, more preferably transfluthrin, in terms of thermal vaporization and diffusion capability, insect killing efficacy, stability, etc. The above pyrethroid insecticidal components may be used alone or in combination. If there are optical or geometrical isomers based on asymmetric carbon for the acid moiety or alcohol moiety of the pyrethroid insecticidal component, these pyrethroid insecticidal component isomers can be used in the present invention.

The content of the pyrethroid insecticidal component in the water-based insecticidal composition is preferably 0.1-3.0 mass%. If the content is less than 0.1 mass%, the insect killing efficacy is likely to decrease. Meanwhile, if the content is more than 3.0 mass%, the properties of the water-based insecticidal composition are likely to be impaired.

The water-based insecticidal composition is a water-based formulation. Therefore, water is used as a solvent for the water-based insecticidal composition. The water-based formulation has a reduced risk of catching fire and is easily made more effective in killing insect pests, compared to oil-based formulations. The water-based formulation is prepared by mixing water with the pyrethroid insecticidal component and a glycol ether compound and/or glycol compound having a boiling point of 150-300°C, preferably 200-260°C. The glycol ether compound and/or the glycol compound have the following actions: (1) solubilizing the pyrethroid insecticidal component; (2) improving the thermal vaporization and diffusion capability; and (3) mediating between the pyrethroid insecticidal component and water so that the three components are thermally vaporized and diffused while the ratio of the three components is maintained constant. Furthermore, it is recognized that the glycol ether compound and/or the glycol compound act as an "efficacy enhancer" for pyrethroid-susceptible insect pests, and have the effect of suppressing the decrease of the insect killing efficacy for insect pests having reduced susceptibility.

The content of the glycol ether compound and/or glycol compound in the water-based insecticidal composition is preferably 10-70 mass%, more preferably 30-60 mass%. If the content is less than 10 mass%, not only is it difficult to prepare a water-based formulation of the water-based insecticidal composition, but also the action of the water-based insecticidal composition as an efficacy enhancer, and the effect of suppressing the decrease of the insect killing efficacy, are poor. Meanwhile, if the content is more than 70 mass%, not only is the insect killing efficacy no longer enhanced, but also the risk of catching fire increases, and therefore, the advantage of being a water-based formulation is likely to be impaired.

Examples of the glycol ether compound and/or glycol compound include diethylene glycol monoethyl ether (boiling point: 202°C), diethylene glycol monoisopropyl ether (boiling point: 207°C, hereinafter referred to as "DEMIP"), diethylene glycol monobutyl ether (boiling point: 231°C, hereinafter referred to as "DEMB"), diethylene glycol monoisobutyl ether (boiling point: 220°C, hereinafter referred to as "DEMIB"), diethylene glycol monohexyl ether (boiling point: 259°C, hereinafter referred to as "DEMH"), diethylene glycol mono2-ethylhexyl ether (boiling point: 272°C), diethylene glycol monophenyl ether (boiling point: 283°C), triethylene glycol monomethyl ether (boiling point: 249°C), propylene glycol mono-tertiary butyl ether (boiling point: 151°C), dipropylene glycol monomethyl ether (boiling point: 188°C), dipropylene glycol monopropyl ether (boiling point: 210°C, hereinafter referred to as "DPMP"), 3-methoxy-1,2-propanediol (boiling point: 220°C), and hexylene glycol (boiling point: 197°C, hereinafter referred to as "HG"). Of them, diethylene glycol monoethyl ether, diethylene glycol monoisopropyl ether, diethylene glycol monobutyl ether, diethylene glycol monoisobutyl ether, and diethylene glycol monohexyl ether are preferable, more preferably diethylene glycol monobutyl ether. The above glycol ether compounds and/or glycol compounds may be used alone or in combination.

Other various components may be added to the water-based insecticidal composition. For example, other pyrethroid insecticidal components, such as allethrin and prallethrin, repellent components, such as DEET, terpene compounds, natural essential oils, and aroma chemicals, antibacterial agents, antifungal agents, stabilizers, such as dibutylhydroxytoluene (BHT) and methyl parahydroxybenzoate, pH adjusting agents, coloring agents, deodorants, such as tea extracts and tea leaf dry distilled solutions, etc., may be added as appropriate. During the preparation of the water-based insecticidal composition, lower alcohols such as ethanol and isopropanol, hydrocarbon solvents such as kerosene, ester or ether solvents, solubilizers, and dispersants may be used, as appropriate, in an amount that does not impair the advantage of being a water-based formulation, in addition to water. The water-based insecticidal composition thus prepared is loaded in a container body (not shown) equipped with a thermal vaporization/diffusion absorbent wick, so that the insect pest control product (liquid mosquito killer) of the present invention is constructed.

In the insect pest control product of the present invention, the thermal vaporization/diffusion absorbent wick has a pyrethroid insecticidal component/(glycol ether compound and/or glycol compound) condensation ratio (hereinafter simply referred to as a "insecticidal component/compound condensation ratio") P₁/P₂ that falls within an appropriate range. As used herein, the term "insecticidal component/compound condensation ratio (P₁/P₂)" with respect to a thermal vaporization/diffusion absorbent wick refers to a parameter related to the vaporization and diffusion capability of a chemical agent from the absorbent wick, which is specified as follows. Initially, 45 mL of the water-based insecticidal composition is loaded into a container. Next, a thermal vaporization/diffusion absorbent wick having a height of approximately 70 mm is put into the container through a stopper. Thus, a water-based liquid mosquito killer that will last for 60 days is prepared. Here, the weight ratio (P₂) of the pyrethroid insecticidal component and the glycol ether compound and/or glycol compound contained in the water-based insecticidal composition before use is previously obtained. Next, the water-based liquid mosquito killer is attached to a chemical agent vaporization and diffusion device, and thermal vaporization and diffusion are performed at 100-130°C. The passage of an electric current (heating) is stopped 2-0 days before the end of duration of use (at day 60 if the duration of use exceeds 60 days), and the thermal vaporization/diffusion absorbent wick is removed within the following two days. The thermal vaporization/diffusion absorbent wick is divided into three equal-length segments, i.e., an upper segment, a middle segment, and a lower segment. The contents of the pyrethroid insecticidal component and the glycol ether compound and/or glycol compound contained in the upper segment are each analyzed to obtain the weight ratio (P₁) of the pyrethroid insecticidal component and the glycol ether compound and/or glycol compound contained in the thermal vaporization/diffusion absorbent wick after use, i.e., after the end of the passage of an electric current. The ratio (P₁/P₂) of P₁ to P₂ is calculated. The insecticidal component/compound condensation ratio in the thermal vaporization/diffusion absorbent wick is defined as this ratio.

Liquid mosquito killers are configured such that a thermal vaporization/diffusion absorbent wick is put in a water-based insecticidal composition, and the water-based insecticidal composition is absorbed and transported to a top portion of the thermal vaporization/diffusion absorbent wick, where the insecticidal composition is heated at 60-130°C, so that a pyrethroid insecticidal component contained in the insecticidal composition is vaporized and diffused into the atmosphere, whereby insect pests are controlled. As described in the "TECHNICAL PROBLEM" section, an ideal water-based insecticidal composition used in a liquid mosquito killer is such that when the thermal vaporization/diffusion absorbent wick is put in the water-based insecticidal composition, the water-based insecticidal composition rises through the thermal vaporization/diffusion absorbent wick while maintaining the original ratio of the components of the liquid composition, and is then vaporized and diffused from the top portion of the thermal vaporization/diffusion absorbent wick into the air. However, there is a difference in vaporization and diffusion capability between the components contained in the water-based insecticidal composition, so that a component having a lower vaporization and diffusion capability may generally have a higher concentration at the top portion of the thermal vaporization/diffusion absorbent wick, and therefore, the composition fraction of that component may relatively increase. As described above, such a phenomenon depends on the properties of a material for the thermal vaporization/diffusion absorbent wick, the affinity between the thermal vaporization/diffusion absorbent wick and the water-based insecticidal composition, the liquid absorption speed of the thermal vaporization/diffusion absorbent wick, etc., and particularly easily occurs for the water-based insecticidal composition. This has been further studied by the present inventors to find that even if a certain component is present at a somewhat high concentration in an upper portion of the thermal vaporization/diffusion absorbent wick, then when that concentration falls within a predetermined range, the entire thermal vaporization/diffusion system is not substantially affected. If thermal vaporization and diffusion is performed using a water-based insecticidal composition containing a compound having a vapor pressure of 2 × 10⁻⁴ to 1 × 10⁻² mmHg at 30°C, selected as a pyrethroid insecticidal component, and a glycol ether compound and/or glycol compound having a boiling point of 150-300°C, then when a thermal vaporization/diffusion absorbent wick that has an above-defined insecticidal component/compound condensation ratio (P₁/P₂) within the range of 1.2-6.0 is used, even the water-based formulation can have satisfactory vaporization and diffusion performance and practical insect killing efficacy. In other words, it was demonstrated that, by combining a thermal vaporization/diffusion absorbent wick satisfying the above range and a water-based insecticidal composition satisfying the above range, the performance of the thermal vaporization/diffusion absorbent wick can be maximized.

A typical container for accommodating the water-based insecticidal composition is a plastic container of a polyolefin, such as polypropylene, a polyester, polyvinyl chloride, etc. The absorbent wick is attached to an upper portion of the chemical liquid container through a stopper. In the case of a water-based formulation, a material for the chemical liquid container is preferably a polyolefin plastic, such as polypropylene, taking into consideration the physical properties of the glycol ether compound and/or glycol compound.

Incidentally, thermal vaporization/diffusion absorbent wicks for liquid mosquito killers are typically roughly divided into baked wicks, braided wicks, and bound wicks. In the present invention, baked wicks or braided wicks are preferably used. A case where a baked wick or a braided wick is used as the thermal vaporization/diffusion absorbent wick will be described below. Note that any materials that are stable with respect to the water-based insecticidal composition containing a pyrethroid insecticidal component, and can absorb aqueous solution through capillary action, can be used for the thermal vaporization/diffusion absorbent wick.

Baked wicks are obtained by baking a mixture containing (a) an inorganic powder, (b) an inorganic binder, and (c) an organic substance (carbonaceous powders, organic binders, etc.) at 600-2000°C. A baked wick that contains only a small amount of the components (b) and (c), i.e. contains almost only the inorganic powder (a), is typically and mostly referred to as a "porous ceramic wick."

Examples of the inorganic powder include mica, alumina, silica, talc, mullite, cordierite, and zirconia. Of them, mica is particularly preferable because it can impart relatively uniform fine pores to a thermal vaporization/diffusion absorbent wick for a liquid mosquito killer. The above inorganic powders may be used alone or in combination. The content of the inorganic powder in the thermal vaporization/diffusion absorbent wick is preferably 10-90 mass%, more preferably 30-70 mass%. The inorganic powder is preferably fine powder of 50 mesh or finer in terms of physical properties such as external appearance, liquid absorption capability, and strength, unless the process of manufacturing the thermal vaporization/diffusion absorbent wick includes pulverization.

Examples of the inorganic binder include clays, such as kaolinite, bentonite, and halloysite, tar pitch, and water glass. Of them, clays are preferable because they have good binding capability. The above inorganic binders may be used alone or in combination. The content of the inorganic binder in the thermal vaporization/diffusion absorbent wick is preferably 5-50 mass%, more preferably 10-40 mass%. The inorganic binders have poor binding action at room temperature, and acquire sufficient binding action by being baked at 600-2000°C, so that they can be preferably used in the thermal vaporization/diffusion absorbent wick.

Examples of the organic substance include carbonaceous powders, such as graphite, carbon black, activated carbon, charcoal, and coke, or organic binders, such as carboxymethyl cellulose (CMC), acrylic resins, and polyolefin resins. Of them, graphite is preferable because it has a relatively uniform shape and contains less impurities. By adding a carbonaceous powder, such as graphite, to the thermal vaporization/diffusion absorbent wick, the external appearance, color, liquid absorption capability, strength, etc., thereof can be improved. The above carbonaceous powders or organic binders may be used alone or in combination. The content of the organic substance in the thermal vaporization/diffusion absorbent wick is preferably 5-40 mass%. If the content is within this range, the generation of carbon monoxide or carbon dioxide during baking of the thermal vaporization/diffusion absorbent wick can produce continuous air holes in the thermal vaporization/diffusion absorbent wick, to form a structure that is sufficiently porous to exert liquid absorption performance through capillary action.

Note that, in addition to the above substances, the thermal vaporization/diffusion absorbent wick may additionally contain, as appropriate, a preservative, and an antioxidant, such as 4,4'-methylene bis(2-methyl-6-t-butylphenol) or stearyl-β-(3,5-di-t-butyl-9-hydroxyphenyl)propionate.

Braided wicks are typically obtained by covering the outer peripheral surface of a core member with a sheath material for absorbing and vaporizing/diffusing a water-based insecticidal composition, where the sheath material is formed as an aggregation of at least one fiber selected from natural fibers, synthetic fibers, and inorganic fibers. In braided wicks, the core member has the function of keeping the shape of the thermal vaporization/diffusion absorbent wick. The materials for the core member do not necessarily need to have the function of absorbing a water-based insecticidal composition. The core member may be made of, for example, a thermoplastic synthetic resin and/or thermosetting synthetic resin that can withstand temperatures of 130°C or more. Note that, in order to enhance the shape retaining function, the thermoplastic synthetic resin and/or thermosetting synthetic resin of the core member may be reinforced using a fibrous reinforcing material such as glass fiber, ceramic fiber, or carbon fiber, a powder reinforcing material such as silica, alumina, or titanium oxide, which are called a glass powder or an inorganic filler, etc.

The sheath material is typically formed as an aggregation of fibers. The aggregation of fibers includes one or more kinds of fibers. Examples of the fibers include natural fibers, such as cotton, synthetic fibers, such as polypropylene, polyesters, nylons, and aramids, and inorganic fibers, such as glass fiber and carbon fiber. Synthetic fibers that can withstand temperatures of 130°C or more, such as polypropylene, polyesters, nylons, and aramids, are preferable. Such a fiber aggregation is typically formed of a fiber material in the form of braid, woven fabric, knitted fabric, felt, nonwoven fabric, etc. In this case, the fiber material may be treated with a surfactant so that the liquid absorption speed is adjusted. Furthermore, the surface of the sheath material may be covered with a varnish, etc., or may be treated so that a function such as hydrophilicity is imparted thereto.

The thermal vaporization/diffusion absorbent wick thus obtained is applied to a liquid product in which the water-based insecticidal composition is thermally vaporized and diffused through the thermal vaporization/diffusion absorbent wick. Specifically, the water-based insecticidal composition is accommodated in a chemical liquid container, and a lower portion of the thermal vaporization/diffusion absorbent wick is put into the water-based insecticidal composition through a stopper. Thereafter, the water-based insecticidal composition in the chemical liquid container is transported to a top portion of the thermal vaporization/diffusion absorbent wick, and is heated to 60-130°C by a heat generator provided at the top portion of the thermal vaporization/diffusion device, to be vaporized and diffused into the atmosphere. The thermal vaporization/diffusion absorbent wick faces a hollow cylindrical heat dissipation tube which is included in the heat generator with a space therebetween. Therefore, the desired surface temperature (e.g., 60-130°C) of the top portion of the thermal vaporization/diffusion absorbent wick is achieved by adjusting the heat generator to a higher temperature (e.g., 80-150°C). If the heating temperature of the water-based insecticidal composition is excessively high, the water-based insecticidal composition is likely to be quickly vaporized and diffused, or the water-based insecticidal composition is likely to undergo pyrolysis or polymerization, leading to the production of a high-boiling-point substance on the surface of the thermal vaporization/diffusion absorbent wick, which may be accumulated to clog the thermal vaporization/diffusion absorbent wick. Meanwhile, if the heating temperature is excessively low, the water-based insecticidal composition has difficulty in vaporizing and diffusing, so that sufficient insect control performance cannot be achieved.

The thermal vaporization/diffusion device used as the insect pest control product of the present invention may be provided with various functions and members similar to those of conventional devices in addition to the above heat generator. For safety, a protective cap is provided over the heat generator. The protective cap has an opening at a center portion thereof. The size and shape of the opening may be arbitrarily determined as long as the liquid formulation vaporized and diffused does not excessively condense or adhere to the protective cap or the device. For example, to provide a cylindrical vaporization/diffusion tube having an inner diameter of 10-30 mm, hanging vertically from near the opening, is effective. In this case, the distance between the lower end of the vaporization/diffusion tube and the top surface of the heat generator is preferably typically within the range of 1-5 mm in terms of the heat resistance and vaporization/diffusion performance of the vaporization/diffusion tube. The insect pest control product of the present invention may be additionally provided, as appropriate, with a power supply cord, on-off operation switch, pilot light, etc., which are coupled to the heat generator.

The thermal vaporization/diffusion method of using the thermal vaporization/diffusion absorbent wick of the present invention has practical insect killing efficacy, in indoor spaces, such as living rooms, lounges, and bedrooms, on not only strains that are susceptible to pyrethroids, but also strains that have reduced susceptibility, of Culex (Culex pipiens pallens, Culex tritaeniorhynchus, Culex pipiens quinquefasciatus, Culex pipiens molestus, etc.), Aedes (Aedes aegypti, Aedes albopictus, etc.), Chironomidae, etc., and other flying insect pests such as houseflies, drain flies, phorid flies, horseflies, black flies, and biting midges, and therefore, is considerably useful.

### Examples

Next, the insect pest control product and insect pest control method of the present invention will be described in greater detail by way of specific examples.

### (Example 1)

A water-based insecticidal composition was prepared by mixing 0.9 mass% of transfluthrin, 50 mass% of diethylene glycol monobutyl ether (DEMB), 0.1 mass% of dibutylhydroxytoluene (BHT) as a stabilizer, and 49 mass% of purified water.

A thermal vaporization/diffusion absorbent wick (a round bar having a diameter of 7 mm and a length of 66 mm) was prepared as follows: water was added to a mixture of 52 mass% of mica powder as the inorganic powder, 33 mass% of kaolinite powder as the inorganic binder, 10 mass% of graphite as the organic substance, 3 mass% of carboxymethyl cellulose as the organic binder, and 2 mass% of starch, followed by kneading, the kneaded mixture was extruded while pressure was applied thereto, followed by air drying and then baking at 1100°C.

Forty-five milliliters of the water-based insecticidal composition was loaded in a plastic container, and the thermal vaporization/diffusion absorbent wick was put into the container through a stopper. The container was attached to a thermal vaporization/diffusion device (e.g., a device disclosed in Japanese Patent No. 2926172, etc., equipped with a hollow cylindrical heat dissipation tube is disposed around a top portion of the absorbent wick (inner diameter: 10 mm, height: 10 mm, and surface temperature: 137°C)). Thus, the insect pest control product of Example 1 was constructed.

The insect pest control product of Example 1 was placed at the center of a room having an area of 6 Jyos (Jyo is a Japanese unit of area: 1 Jyo is equal to approximately 1.7 m²) (25 m³), and was used while an electric current was passed through the heat generator for 12 hours per day. For 60 days (approximately 700 hours), no mosquito biting was observed.

The insecticidal component/compound condensation ratio (P₁/P₂) in the thermal vaporization/diffusion absorbent wick that was measured immediately after the end of the passage of an electric current was 1.8.

### (Examples 2-9 and Comparative Examples 1-7)

Water-based insecticidal compositions and thermal vaporization/diffusion absorbent wicks used in Examples 2-9 were prepared in a manner similar to that for Example 1, and were loaded into respective thermal vaporization/diffusion devices to construct respective insect pest control products of Examples 2-9. Each insect pest control product was measured and tested for (1) - (3) described below. For comparison, insect pest control products of Comparative Examples 1-7 were similarly measured and tested. The formulations of the chemical liquids and the component contents of the thermal vaporization/diffusion absorbent wicks, of the examples and the comparative examples, are shown in Table 1, including those of Example 1.

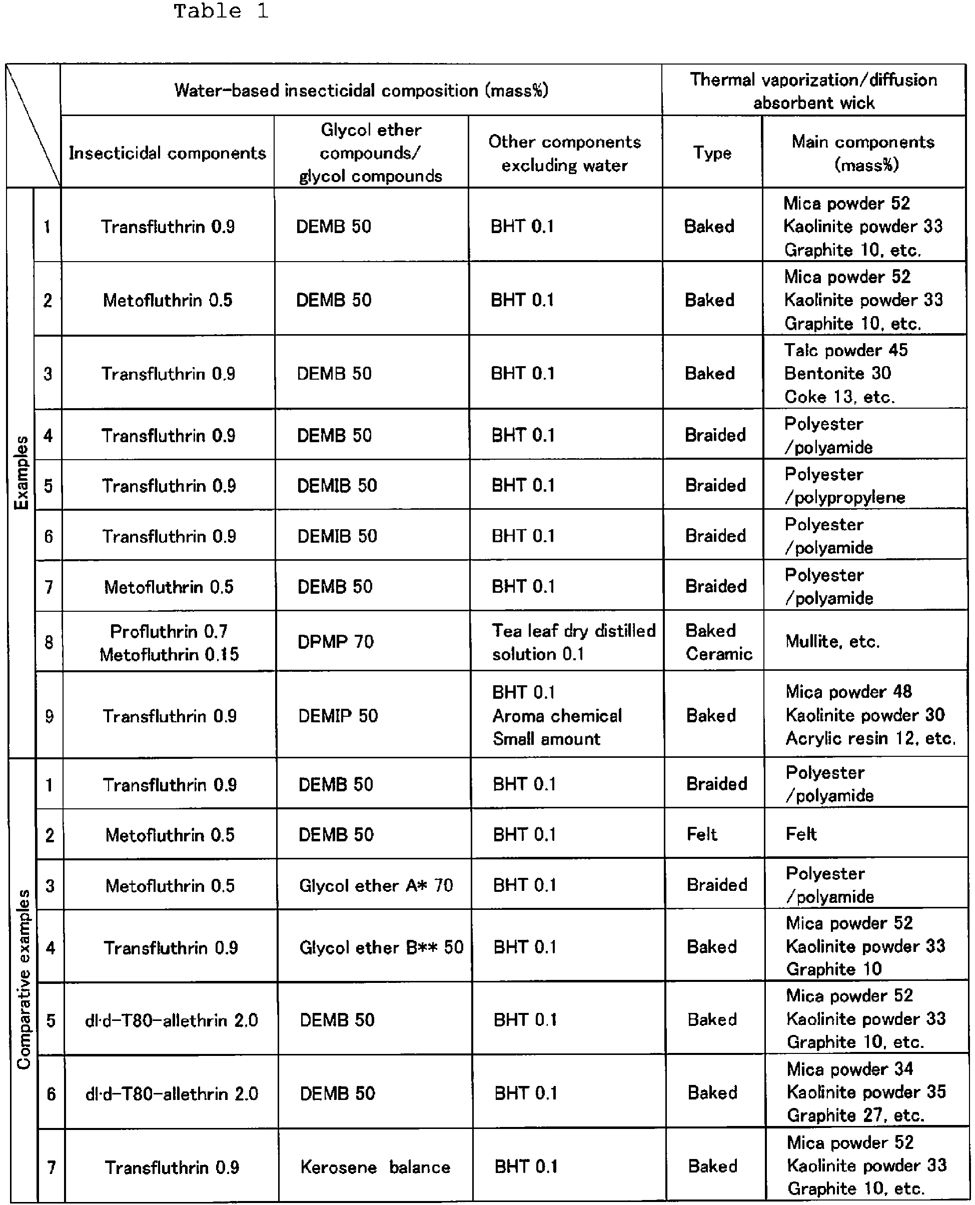

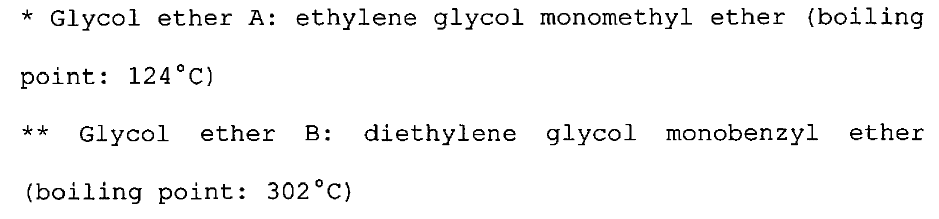

### (1) Vaporization and Diffusion Performance

A thermal vaporization/diffusion device to be tested was placed at the center of a 6-Jyo room (25 m³), and was heated through the passage of an electric current. The insecticidal component was trapped using a silica gel-filled column, extracted using acetone, and analyzed by gas chromatography, at predetermined time intervals, so that the amount of the insecticidal component vaporized and diffused per unit time was obtained. The vaporization and diffusion performance was determined at an early part of the period of use (day 2), a middle part of the period of use (approximately a half past the expiration date), and a late part of the period of use (several days before the expiration date).

### (2) Insect Killing Efficacy Test

Two plastic cylinders each having an inner diameter of 20 cm and a height of 43 cm were put on top of each other. Another cylinder having an inner diameter of 20 cm and a height of 20 cm (insects to be tested were to be placed) was put on top of the stack of the two cylinders with a 16-mesh metal mesh being interposed therebetween. Still another cylinder having the same inner diameter and a height of 20 cm was put on top of the third cylinder with a 16-mesh metal mesh being interposed therebetween. The stack of the four cylinders was placed on a circular plate provided on a table with a rubber gasket being interposed between the cylinder stack and the circular plate. The circular plate had a 5-cm circular hole at the center thereof. Each insect pest control product was placed on the circular hole, and heated through the passage of an electric current. After four hours of the passage of an electric current, approximately 20 adult female Culex pipiens pallens mosquitoes (insects to be tested) were released in the second uppermost cylinder. The number of tested insects which fell down to be flat on their back as time passed was counted to calculate the KT₅₀ value. After 20 minutes of exposure, all of the tested insects were collected. The fatality rate of the insects was investigated 24 hours later. The insect killing efficacy test was conducted at an early part of the period of use (day 2) and a late part of the period of use (several days before the expiration date).

### (3) Insecticidal Component/Compound Condensation Ratio

The thermal vaporization/diffusion absorbent wick was removed within two days after the end of the passage of an electric current. The thermal vaporization/diffusion absorbent wick was divided into three equal-length segments, i.e., an upper segment, a middle segment, and a lower segment. The contents of the pyrethroid insecticidal component and the glycol ether compound and/or glycol compound contained in the upper segment were analyzed to obtain the weight ratio (P₁) of the pyrethroid insecticidal component and the glycol ether compound and/or glycol compound contained in the thermal vaporization/diffusion absorbent wick after use, i.e., after the end of the passage of an electric current. This was divided by the weight ratio (P₂) of the pyrethroid insecticidal component and the glycol ether compound and/or glycol compound contained in the water-based insecticidal composition before use, which had been previously calculated, to obtain the insecticidal component/compound condensation ratio (P₁/P₂) of the thermal vaporization/diffusion absorbent wick.

The test results of the examples and the comparative examples are shown in Table 2.

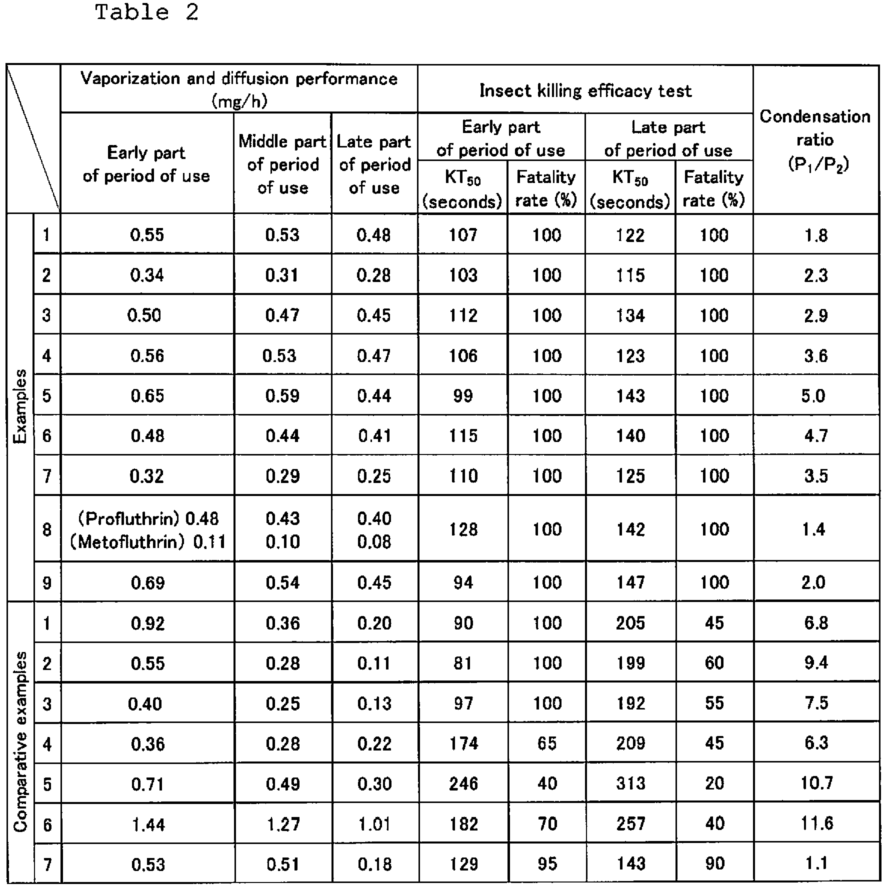

The thermal vaporization/diffusion absorbent wicks of Examples 1-9 were applied to a water-based insecticidal composition containing a pyrethroid insecticidal component having a vapor pressure of 2 × 10⁻⁴ to 1 × 10⁻² mmHg at 30°C, a glycol ether compound and/or glycol compound having a boiling point of 150-300°C, and water. According to the test results, it was verified that the thermal vaporization/diffusion absorbent wicks have an insecticidal component/compound condensation ratio (P₁/P₂) within the range of 1.2-6.0, and therefore, have stable vaporization and diffusion performance and good insect killing efficacy no matter whether they are a baked wick or a braided wick, and are considerably effective in controlling flying insect pests, particularly mosquitoes. Note that, for example, as can be seen from comparison between Example 4 and Example 6, the glycol ether compound and/or the glycol compound are preferably diethylene glycol monobutyl ether, which is a glycol ether compound.

In contrast to this, in Comparative Examples 1 and 2, although the predetermined pyrethroid insecticidal component and glycol ether compound and/or glycol compound were used, the insecticidal component/compound condensation ratios (P₁/P₂) of the thermal vaporization/diffusion absorbent wicks did not fall within the range of 1.2-6.0. In this case, the amount of the insecticidal component vaporized and diffused tended to be initially great, and thereafter, significantly decrease. Therefore, a sufficient insect killing efficacy to achieve the object of the present invention was not obtained. Furthermore, in Comparative Examples 3 and 4, in which the boiling points of the glycol ether compound and/or glycol compound do not fall within the predetermined range, and in Comparative Examples 5 and 6, in which the vapor pressure of the pyrethroid insecticidal component does not fall within the predetermined range, it was difficult to satisfy the specification of the insecticidal component/compound condensation ratios (P₁/P₂). Furthermore, the oil-based formulation of the composition of Comparative Example 7 had comparable vaporization and diffusion performance and insect killing efficacy, and had the risk of catching fire. Note that the condensation ratio in Comparative Example 7 is an insecticidal component/kerosene condensation ratio in the thermal vaporization/diffusion absorbent wick.

### INDUSTRIAL APPLICABILITY

The present invention is applicable as an insect pest control product for humans and pets, and has other applications, such as insecticidal, acaricidal, sterilizing, antimicrobial, deodorizing, and antibromic applications.

## Claims

1. An insect pest control product comprising a thermal vaporization/diffusion absorbent wick for vaporizing and diffusing a water-based insecticidal composition containing a pyrethroid insecticidal component having a vapor pressure of 2 × 10⁻⁴ to 1 × 10⁻² mmHg at 30°C, a glycol ether compound and/or glycol compound having a boiling point of 150-300°C, and water, **characterized in that**
the thermal vaporization/diffusion absorbent wick has an insecticidal component/compound condensation ratio (P₁/P₂) within the range of 1.2-6.0, where the insecticidal component/compound condensation ratio (P₁/P₂) is calculated from a weight ratio (P₁) of the pyrethroid insecticidal component and the glycol ether compound and/or glycol compound contained in the thermal vaporization/diffusion absorbent wick after use, and a weight ratio (P₂) of the pyrethroid insecticidal component and the glycol ether compound and/or glycol compound contained in the water-based insecticidal composition before use.

2. The insect pest control product of claim 1, **characterized in that**
the pyrethroid insecticidal component is at least one selected from the group consisting of transfluthrin, metofluthrin, and profluthrin.

3. The insect pest control product of claim 1 or 2, **characterized in that**
the glycol ether compound is a diethylene glycol alkyl ether.

4. The insect pest control product of claim 3, **characterized in that**
the diethylene glycol alkyl ether is diethylene glycol monobutyl ether.

5. The insect pest control product of any one of claims 1-4, **characterized in that**
the thermal vaporization/diffusion absorbent wick is a baked wick or a braided wick.

6. The insect pest control product of claim 5, **characterized in that**
the baked wick contains, as raw materials, an inorganic powder, an inorganic binder, and an organic substance.

7. The insect pest control product of claim 5, **characterized in that**
the braided wick has a core member, and a sheath material covering an outer peripheral surface of the core member, and
the sheath material contains at least one fiber selected from the group consisting of natural fibers, synthetic fibers, and inorganic fibers.

8. An insect pest control method of using the insect pest control product of any one of claims 1-7, **characterized in that** the method comprises:
putting the thermal vaporization/diffusion absorbent wick in the water-based insecticidal composition so that the water-based insecticidal composition is absorbed and transported to a top portion of the thermal vaporization/diffusion absorbent wick, and heating the top portion at 60-130°C so that the pyrethroid insecticidal component is vaporized and diffused into the atmosphere.
